# EUROPEAN PATENT APPLICATION

(11) **EP 3 081 200 A1**
(43) Date of publication of application: **19.10.2016**
(21) Application number: 15173399.5
(22) Date of filing: 23.06.2015
(51) Int. Cl.: A61F 13/47, A61F 13/84, A61F 13/15

(54) **METHOD OF USING ABSORBENT SHEETS TO COLLECT SWEAT FROM BODY CREASES**

(30) Priority: 14.04.2015 US 201514685986
(71) Applicant: Burgess, Andrea, Wilmington, DE 19805 (US)
(72) Inventor: Burgess, Andrea, Wilmington, DE 19805 (US)
(74) Representative: Hocking, Adrian Niall

(57) **Abstract**

A method of using absorbent sheets to collect sweat from body creases includes utilizing a plurality of absorbent sheets (1) positioned into a high-perspiration body crease (2) and attached to an external body surface (3). Once the plurality of absorbent sheets (1) is positioned within the high-perspiration body crease (2), sweat is collected from the high-perspiration body crease (2) within the plurality of absorbent sheets (1). A fragrant agent (4) and an antimicrobial agent (5) are present in the plurality of absorbent sheets (1) in order to neutralize any odors due to sweat and any potentially harmful microorganisms in sweat. If the plurality of absorbent sheets (1) becomes saturated with secreted sweat, the plurality of absorbent sheets (1) is removed from the high-perspiration body crease (2). The plurality of absorbent sheets (1) may be adapted to allow sweat to be collected from various regions of the body that are susceptible to excessive perspiration.

## Description

The current application is a continuation-in-part and claims priority to a non-provisional application 14/165,526 filed on January 27, 2014.

### FIELD OF THE INVENTION

The present invention relates generally to a method of collecting excessive perspiration in various regions of the body. More specifically, the present invention is a method of using absorbent sheets to collect sweat from body creases.

### BACKGROUND OF THE INVENTION

Excessive perspiration can be a source of discomfort and embarrassment as well as a catalyst for unsanitary conditions. This excessive perspiration may be brought upon by medical conditions such as menopausal hot flashes and hyperhidrosis or simply due to the effects of a hot environment on the body. Being overweight can also cause excessive perspiration in many areas of the body. Excessive perspiration can soil one's clothing as well as any objects that one comes into contact with. In addition to causing discomfort and embarrassment to oneself, excessive perspiration has negative effects on others as well due to the often unsanitary nature of sweat. It is possible to mitigate perspiration by drinking cool beverages, moving to a cooler environment, or lowering the ambient temperature of one's environment. However, these methods are not always effective, particularly if the excessive perspiration is symptomatic of a related medical condition. Another means of attempting to control perspiration is through the use of antiperspirants. However, these may not always be available or effective. The present invention seeks to address the aforementioned issues related to excessive perspiration as well as provide the user with a convenient, straightforward, and readily accessible solution.

The present invention is a method of using absorbent sheets to collect sweat from body creases. Body creases are the most common sources of excessive perspiration due to the enclosed nature of the body creases. The present invention is adaptable to accommodate various body creases and may be utilized in multiple regions of the user's body. In addition to collecting sweat, the present invention utilizes antimicrobial and deodorizing properties in order to maintain the user's sanitation as well as reduce odors produced by excessive perspiration.

### BRIEF DESCRIPTION OF THE DRAWINGS

FIG. 1 is a flowchart illustrating the overall process that is followed by the present invention.
FIG. **2** is a cross-sectional schematic view of the present invention illustrating the fragrant agent impregnated throughout the plurality of absorbent sheets.
FIG. **3** is a cross-sectional schematic view of the present invention illustrating the antimicrobial agent impregnated throughout the plurality of absorbent sheets.
FIG. **4** is a perspective view of the present invention configured into a band.
FIG. **5** is a front view of the present invention in use in between a potbelly and a pelvic region.
FIG. **6** is a front view of the present invention configured into a band, a pad, and a rope.
FIG. **7** is a front view of the present invention in use in a crotch region.
FIG. **8** is a side view of the present invention in use in a crotch region.
FIG. **9** is a front view of the present invention in use between an under-breast and a belly.
FIG. **10** is a side view of the present invention in use within an armpit.
FIG. **11** is a cross-sectional schematic view of the present invention attached to an external body surface by an adhesive.
FIG. **12** is a cross-sectional schematic view of the present invention attached to an external body surface by a layer of clothing.
FIG. **13** is a perspective view of the present invention in a collapsed configuration and held together by a fastener.
FIG. **14** is a perspective view of the present invention in an operative configuration.
FIG. **15** is a cross-sectional schematic view of the plurality of absorbent sheets layered upon each other.

### DETAIL DESCRIPTIONS OF THE INVENTION

All illustrations of the drawings are for the purpose of describing selected versions of the present invention and are not intended to limit the scope of the present invention.

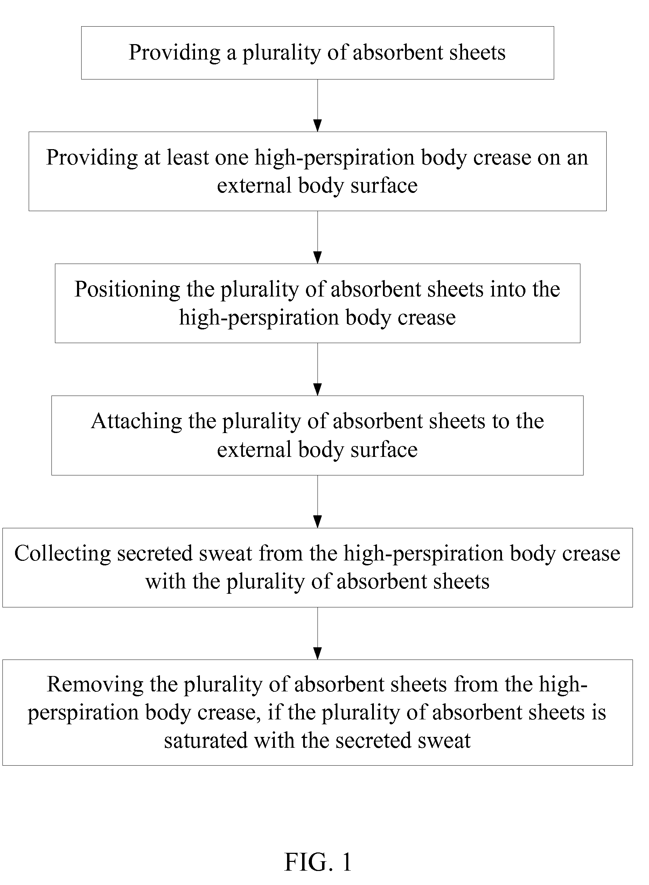

The present invention is a method of using absorbent sheets to collect sweat from body creases. The overall process followed by the present invention is shown in FIG. 1. The plurality of absorbent sheets 1 is capable of absorbing sweat from at least one high-perspiration body crease **2** on an external body surface **3.** This allows the plurality of absorbent sheets **1** to collect sweat in order to minimize contact with the user's clothing as well as any objects that the user comes into contact with. The high-perspiration body crease **2** is a region of the user's body that is susceptible to excessive sweat accumulation. The plurality of absorbent sheets **1** is positioned into the high-perspiration body crease **2** in order to allow sweat secreted through the external body surface **3** to be absorbed directly into the plurality of absorbent sheets **1.** The plurality of absorbent sheets **1** is attached to the external body surface **3,** thereby securing the plurality of absorbent sheets **1** in place within the high-perspiration body crease **2** and preventing the plurality of absorbent sheets **1** from becoming dislodged. The secreted sweat is collected from the high-perspiration body crease **2** with the plurality of absorbent sheets **1** and is prevented from coming into contact with the user's clothing and any objects that the user comes into contact with. The plurality of absorbent sheets **1** is removed from the high-perspiration body crease **2** if the plurality of absorbent sheets **1** is saturated with the secreted sweat. The soiled plurality of absorbent sheets **1** may then be replaced with a fresh plurality of absorbent sheets **1** to collect any further secreted sweat within the high-perspiration body crease **2.** Alternatively, the plurality of absorbent sheets **1** may simply be replaced at any time regardless of the saturation level of the plurality of absorbent sheets **1** and as the user desires in order to improve his or her comfort.

The present invention is not limited with respect to the specific type of material from which the plurality of absorbent sheets **1** is composed. The plurality of absorbent sheets **1** is made of a material selected from the group consisting of toilet paper, paper towels, tissue paper, natural hydrophilic fibers, synthetic hydrophilic fibers, multilayered cellulosic fibers, and a combination thereof. Natural hydrophilic fibers and synthetic hydrophilic fibers are fibers that readily absorb water and as such are highly suitable for absorbing sweat from the high-perspiration body crease **2.** Multilayered cellulosic fibers are highly effective for absorbing sweat as well due to the high absorption of the fibers. Toilet paper, paper towels, and tissue paper are readily accessible household products that are nevertheless effective at absorbing sweat as well.

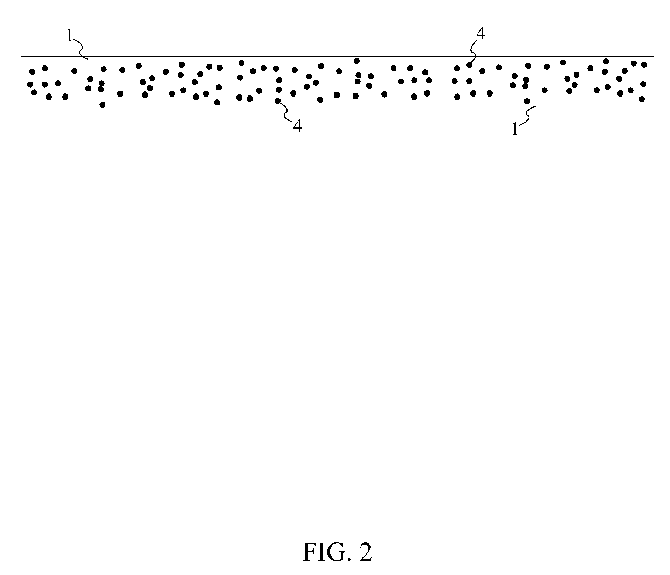

Because of the tendency of secreted sweat to emit an odor, a fragrant agent **4** is impregnated throughout each of the plurality of absorbent sheets **1** as shown in FIG. **2****.** The fragrant agent **4** is able to effectively mask the odor caused by the secreted sweat and prevent the odor from being noticeable in the user's vicinity. The high-perspiration body crease **2** is aromatized by gradually releasing the fragrant agent **4** as the plurality of absorbent sheets **1** collects the secreted sweat. The time-release of the fragrant agent **4** allows the fragrant agent **4** to be engaged simultaneously as the sweat is secreted. The present invention is not limited with respect to the specific fragrant agent **4** utilized. For example, the fragrant agent **4** may be an aromatic oil or essential oil that is impregnated throughout each of the plurality of absorbent sheets **1.** In the preferred embodiment of the present invention, the fragrant agent **4** is hypoallergenic in order to minimize the likelihood of an allergic reaction when the plurality of absorbent sheets **1** is applied to the user's skin.

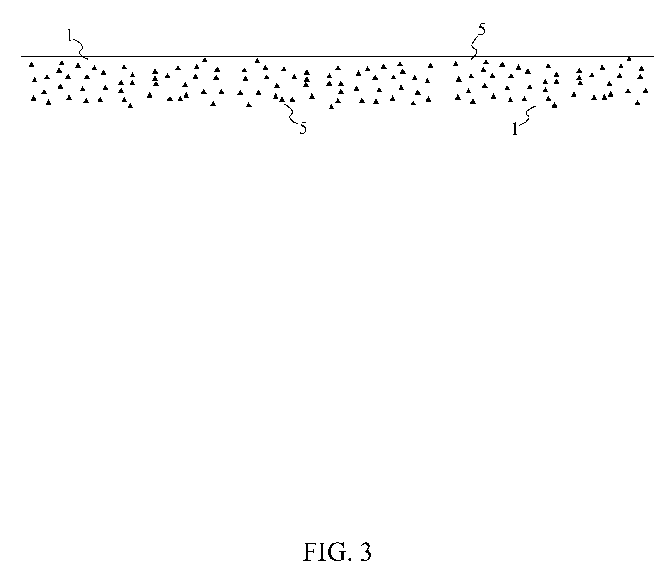

In addition to causing odor, secreted sweat is often unsanitary as well. As such, an antimicrobial agent **5** is impregnated throughout each of the plurality of absorbent sheets **1** as shown in FIG. **3****.** The antimicrobial agent **5** provides antibacterial, antifungal, and other disinfecting properties to the plurality of absorbent sheets **1.** The growth of bacteria and fungi can result in infections, rashes, and other medical conditions. The antimicrobial agent 5 is effective at neutralizing any potentially harmful microorganisms in the secreted sweat that is absorbed into the plurality of absorbent sheets **1.** The high-perspiration body crease **2** is antisepticised by gradually releasing the antimicrobial agent **5** as the plurality of absorbent sheets **1** collects the secreted sweat. This is similar to the release mechanism of the fragrant agent **4** and allows the antimicrobial agent **5** to be engaged and released as the sweat is secreted. In the preferred embodiment of the present invention, the antimicrobial agent **5** is a quaternary ammonium salt. Quaternary ammonium salts are lethal to a wide variety of organisms and as such are highly effective as an antimicrobial agent **5.** Quaternary ammonium compounds are able to disrupt the cell membrane of microorganisms that are found in secreted sweat, effectively neutralizing any potentially harmful microorganisms in the sweat. In order to impregnate the antimicrobial agent **5** throughout each of the plurality of absorbent sheets **1,** a solution of quaternary ammonium salt is applied throughout each of the plurality of absorbent sheets **1** and the solution is then allowed to dry. In the preferred embodiment of the present invention, the antimicrobial agent **5** is hypoallergenic, similar to the fragrant agent **4.**

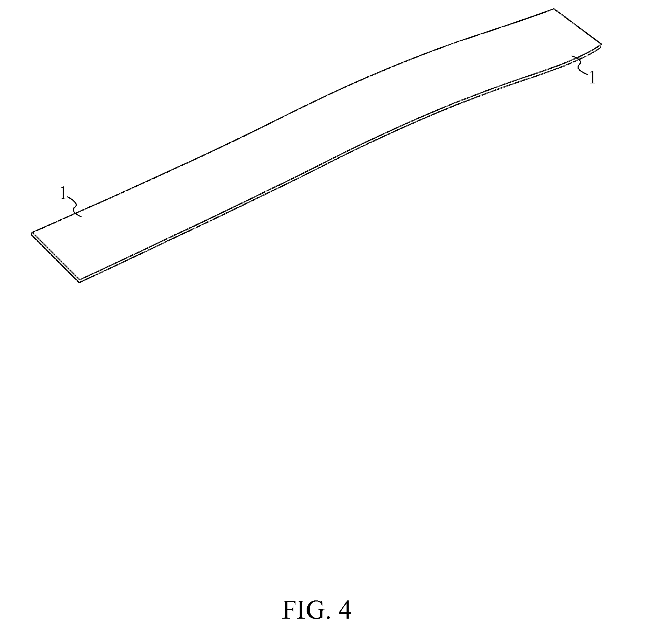

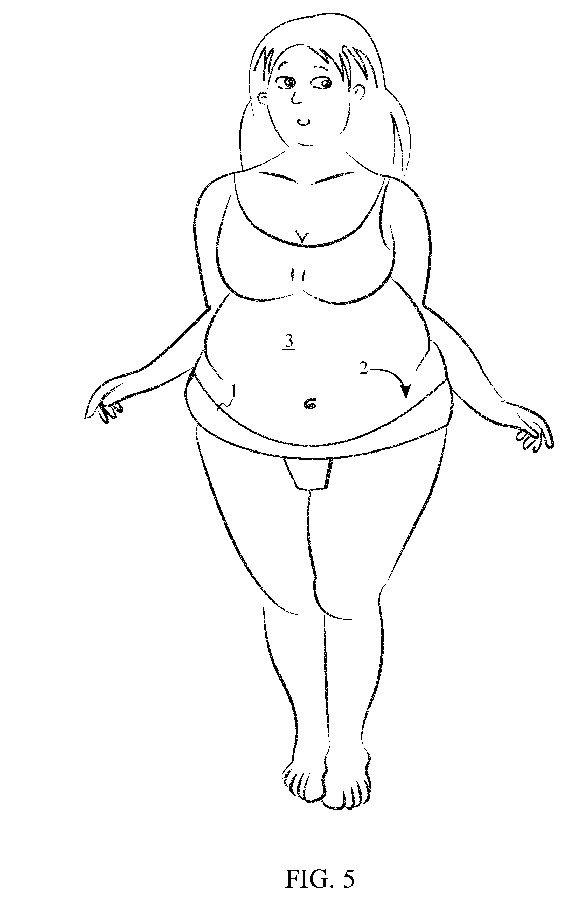

As shown in FIGS. **4-10****,** the present invention is utilized for more than one high-perspiration body crease **2** susceptible to excessive perspiration. One such high-perspiration body crease **2** is between a potbelly and a pelvic region. Because the potbelly is subject to drooping downward toward the pelvic region, the high-perspiration body crease **2** between the potbelly and the pelvic region secretes excessive sweat due to the crease becoming enclosed. In order to collect sweat from the high-perspiration body crease **2,** a portion of the plurality of absorbent sheets **1** is configured into a band as shown in FIG. **4****,** allowing the plurality of absorbent sheets **1** to be wrapped onto the user's body. This band is placed in between the potbelly and the pelvic region as shown in FIG. **5****.** The band is thus placed into contact with the high-perspiration body crease **2** between the potbelly and the pelvic region, allowing secreted sweat to be absorbed into the plurality of absorbent sheets **1.**

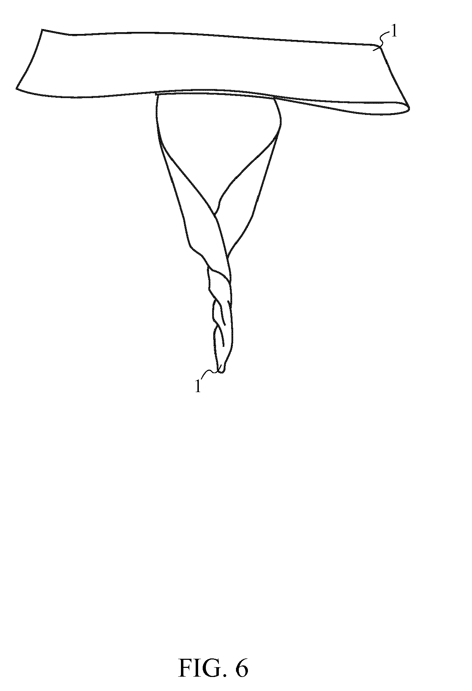

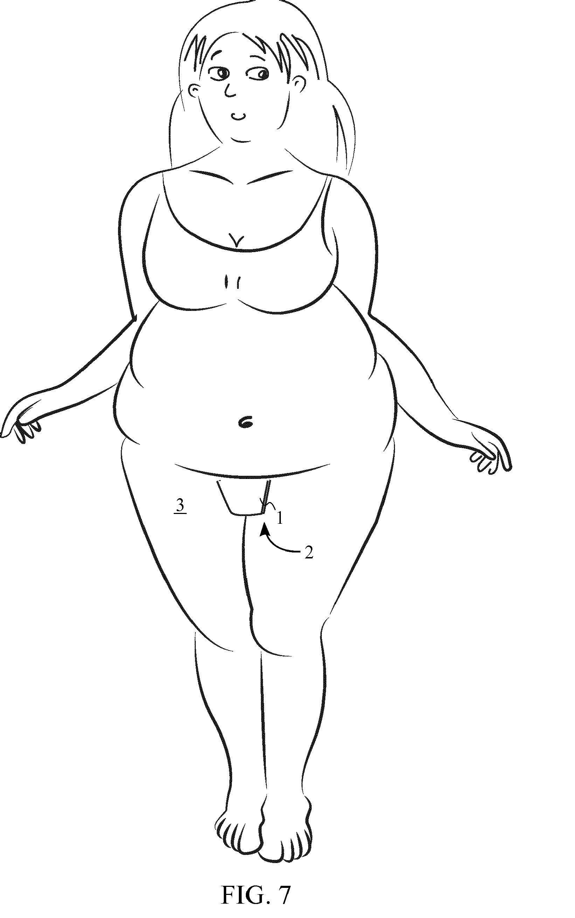

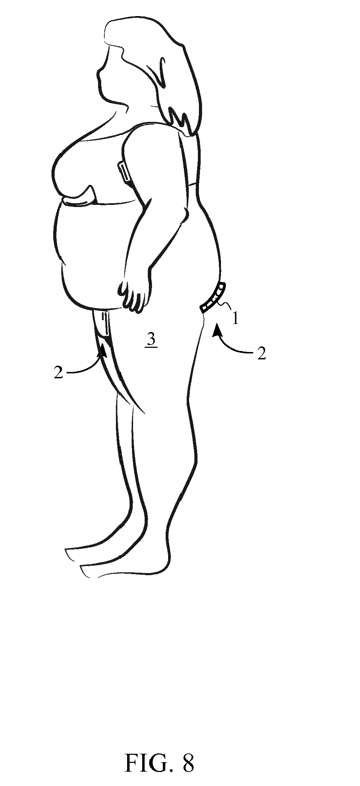

A second high-perspiration body crease **2** is a crotch region that extends from an anterior pelvic region and a posterior pelvic region. As shown in FIG. **6****,** a portion of the plurality of absorbent sheets **1** is configured into a pad while another portion of the plurality of sheets is configured into a rope. The pad is placed onto the crotch region, adjacent to the anterior pelvic region as shown in FIG. **7** and FIG. **8****.** This allows the pad to collect sweat from the vaginal and lower abdominal regions of the user's body. The rope is placed onto the crotch region, adjacent to the posterior pelvic region. The rope is sufficiently thin to be positioned in between the buttocks and absorb sweat from the anal region.

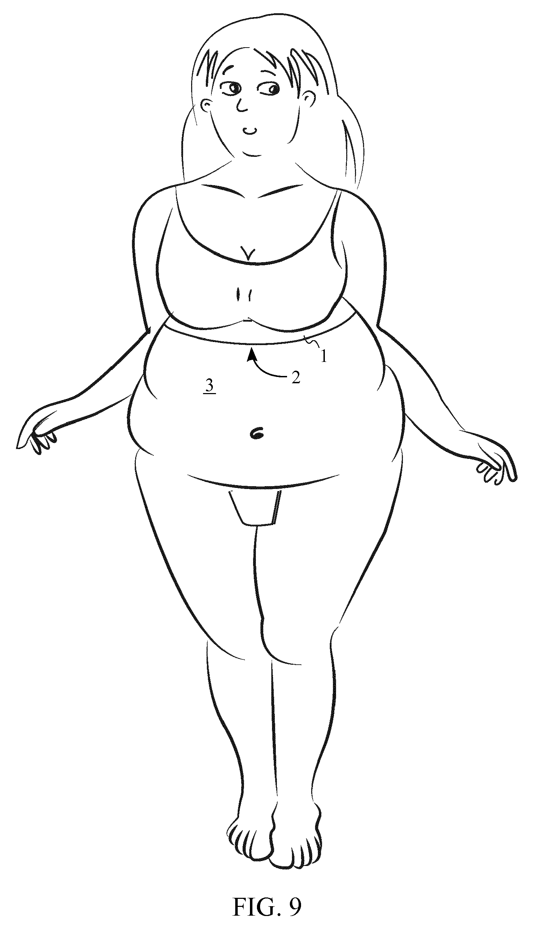

A third high-perspiration body crease **2** is between an under-breast and a belly. This high-perspiration body crease **2** is subject to excessive sweat secretion due to the tendency of the breasts to droop, forming an enclosed space between the under-breast and the belly. Similar to the high-perspiration body crease **2** between the potbelly and the pelvic region, a portion of the plurality of absorbent sheets **1** is configured into a band. The band may be wrapped onto the user's body and placed in between the under-breast and the belly as shown in FIG. **9****.** This allows the plurality of absorbent sheets **1** to absorb sweat secreted between the under-breast and the belly.

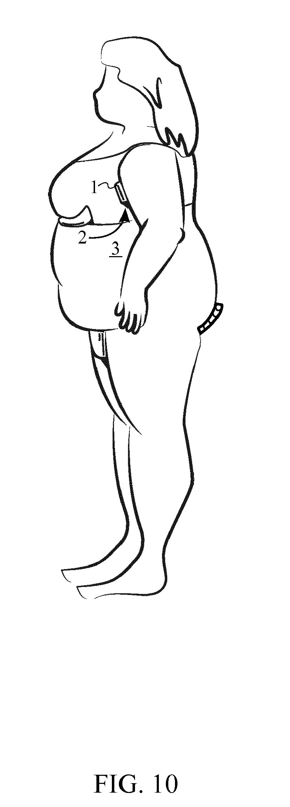

A fourth high-perspiration body crease **2** is the armpit. Similar to the high-perspiration body crease **2** between the anterior pelvic region and the posterior pelvic region, a portion of the plurality of absorbent sheets **1** is configured into a pad. The pad is placed within the armpit as shown in FIG. **10****.** Sweat is often excessively secreted from the armpit due to the enclosed nature of the armpit and the pad is able to absorb the sweat from directly within the high-perspiration body crease **2.**

Because the present invention allows for the collection of sweat secreted in more than one high-perspiration body crease **2,** the plurality of absorbent sheets **1** must be configured accordingly in order to accommodate a particular high-perspiration body crease **2.** The spatial dimensions of the high-perspiration body crease **2** are measured in order to ensure that the plurality of absorbent sheets **1** is able to sufficiently cover and absorb sweat from the high-perspiration body crease **2.** A portion of the plurality of absorbent sheets **1** is proportionately sized in order to match the spatial dimensions of the high-perspiration body crease **2.** The plurality of absorbent sheets **1** is then placed into the high-perspiration body crease **2** and is able to absorb secreted sweat.

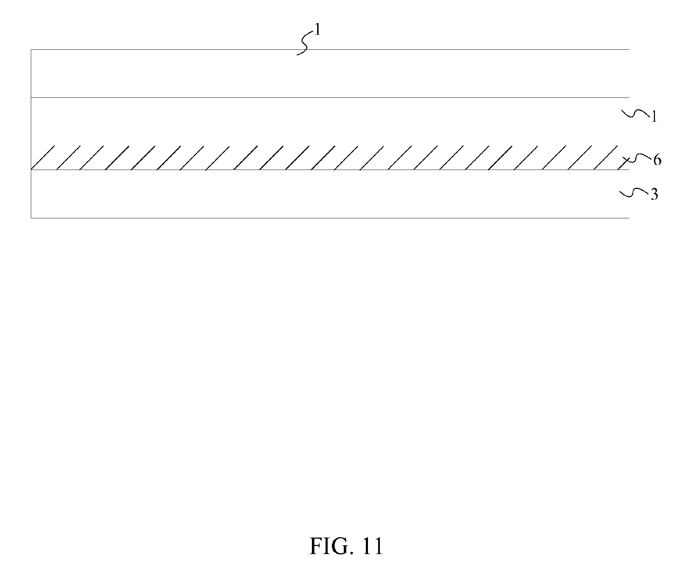

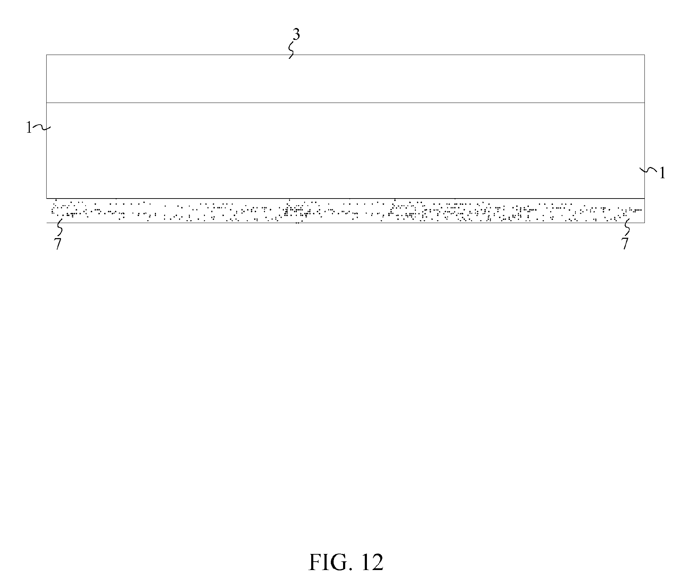

The present invention is not limited with respect to the specific means by which the plurality of absorbent sheets **1** is attached to the external body surface **3.** The plurality of absorbent sheets **1** may be attached to the external body surface **3** by an adhesive **6** as shown in FIG. **11****.** The adhesive **6** allows the plurality of absorbent sheets **1** to be secured directly to the external body surface **3** and additionally prevents the plurality of absorbent sheets **1** from separating from the external body surface **3.** It is important to ensure that the adhesive **6** utilized to attach the plurality of absorbent sheets **1** to the external body surface **3** does not become compromised when exposed to secreted sweat. In an alternative to the adhesive **6,** the plurality of absorbent sheets **1** may be held against the external body surface **3** by pressing the plurality of absorbent sheets **1** against the external body surface **3** with a clothing layer **7** as demonstrated in FIG. **12****.** This allows the user to simply wear the plurality of absorbent sheets **1** underneath his or her clothing. However, the plurality of absorbent sheets **1** is not attached to the external body surface **3** as with the adhesive **6,** but simply pressed against the external body surface **3.** Because the adhesive **6** is placed into direct contact with the user's skin, in the preferred embodiment of the present invention, the adhesive **6** is hypoallergenic.

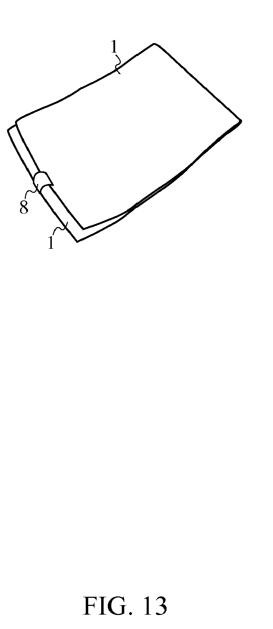

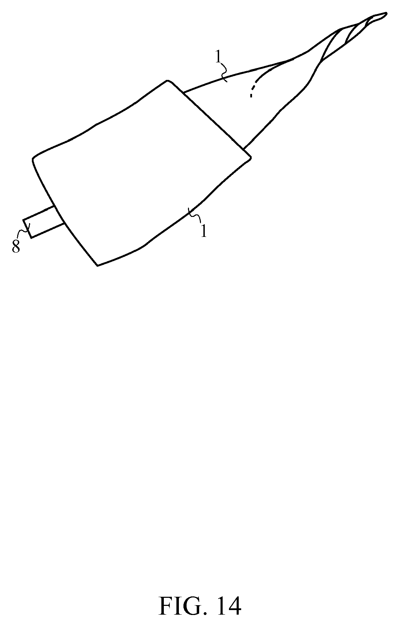

When the plurality of absorbent sheets **1** is not in use, the plurality of absorbent sheets **1** is held in a collapsed configuration while the plurality of absorbent sheets **1** is held in an operative configuration when in use. The present invention is shown in the collapsed configuration in FIG. **13** and the operative configuration in FIG. **4****,** FIG. **6****,** and FIG. **14****.** The collapsed configuration has smaller spatial dimensions than the operative configuration in order to increase the compactness of the plurality of absorbent sheets **1.** The plurality of absorbent sheets **1** is unfolded into the operative configuration. In the preferred embodiment of the present invention, the collapsed configuration is held together by a fastener **8.** The fastener **8** is able to prevent the plurality of absorbent sheets **1** from becoming unraveled from the collapsed configuration. In the preferred embodiment of the present invention, the fastener **8** is hypoallergenic as well, similar to the fragrant agent **4,** the antimicrobial agent **5,** and the adhesive **6.**

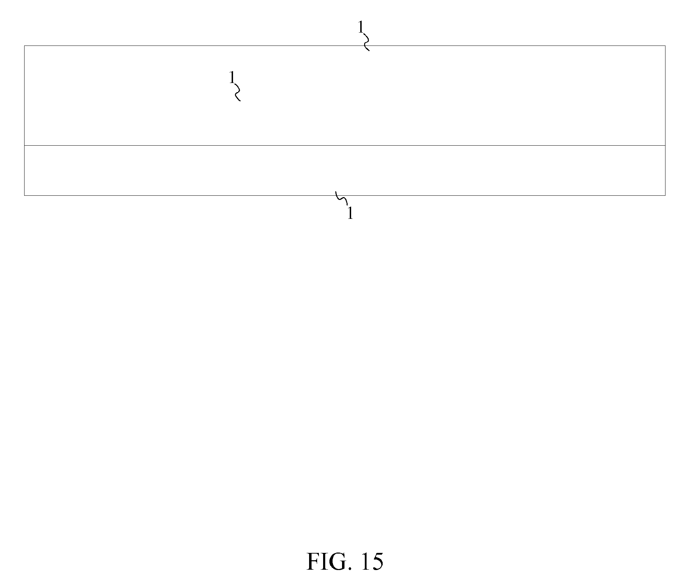

In the preferred embodiment of the present invention, the plurality of absorbent sheets **1** is layered upon each other as shown in FIG. **15** in order to provide multiple layers into which sweat may be absorbed. Multiple layers of the plurality of absorbent sheets **1** further minimizes the likelihood of secreted sweat coming into contact with the user's clothing and nearby objects the user comes into contact with.

Although the present invention has been explained in relation to its preferred embodiment, it is understood that many other possible modifications and variations can be made without departing from the spirit and scope of the present invention as hereinafter claimed.

## Claims

1. A method of using absorbent sheets to collect sweat from body creases, the method comprising the steps of:
providing a plurality of absorbent sheets (1);
providing at least one high-perspiration body crease (2) on an external body surface (3);
positioning the plurality of absorbent sheets (1) into the high-perspiration body crease (2);
attaching the plurality of absorbent sheets (1) to the external body surface (3);
collecting secreted sweat from the high-perspiration body crease (2) with the plurality of absorbent sheets (1); and
removing the plurality of absorbent sheets (1) from the high-perspiration body crease (2),
if the plurality of absorbent sheets (1) is saturated with the secreted sweat.

2. A method as claimed in claim 1, wherein the plurality of absorbent sheets (1) is made of a material selected from the group consisting of: toilet paper, paper towels, tissue paper, natural hydrophilic fibers, synthetic hydrophilic fibers, multilayered cellulosic fibers, and a combination thereof.

3. A method as claimed in claim 1, the method further comprising the steps of:
providing a fragrant agent (4) impregnated throughout each of the plurality of absorbent sheets (1); and
aromatizing the high-perspiration body crease (2) by gradually releasing the fragrant agent (4) as the plurality of absorbent sheets (1) collects the secreted sweat.

4. A method as claimed in claim 1, the method further comprising the steps of:
providing an antimicrobial agent (5) impregnated throughout each of the plurality of absorbent sheets (1); and
antisepticising the high-perspiration body crease (2) by gradually releasing the antimicrobial agent (5) as the plurality of absorbent sheets (1) collects the secreted sweat.

5. A method as claimed in claim 4, wherein the antimicrobial agent (5) is a quaternary ammonium salt.

6. A method as claimed in claim 1, the method further comprising the steps of:
providing a crease between a potbelly and a pelvic region as the high-perspiration body crease (2);
configuring a portion of the plurality of absorbent sheets (1) into a band;
and
placing the band in between the potbelly and the pelvic region.

7. A method as claimed in claim 1, the method further comprising the steps of:
providing a crotch region as the high-perspiration body crease (2), wherein the crotch region extends from an anterior pelvic region and a posterior pelvic region;
configuring a portion of the plurality of absorbent sheets (1) into a pad;
configuring another portion of the plurality of absorbent sheets (1) into a rope;
placing the pad onto the crotch region, adjacent to the anterior pelvic region; and
placing the rope onto the crotch region, adjacent to the posterior pelvic region.

8. A method as claimed in claim 1, the method further comprising the steps of:
providing a crease between an under-breast and a belly as the high-perspiration body crease (2);
configuring a portion of the plurality of absorbent sheets (1) into a band; and
placing the band in between the under-breast and the belly.

9. A method as claimed in claim 1, the method further comprising the steps of:
providing an armpit as the high-perspiration body crease (2);
configuring a portion of the plurality of absorbent sheets (1) into a pad; and
placing the pad within the armpit.

10. A method as claimed in claim 1, the method further comprising the steps of:
measuring spatial dimensions of the high-perspiration body crease (2); and
proportionately sizing a portion of the plurality of absorbent sheets (1) in order to match the spatial dimensions of the high-perspiration body crease (2).

11. A method as claimed in claim 1, wherein the plurality of absorbent sheets (1) is attached to the external body surface (3) by an adhesive (6).

12. A method as claimed in claim 1, wherein the plurality of absorbent sheets (1) is attached to the external body surface (3) by pressing the plurality of absorbent sheets (1) against the external body surface (3) with a clothing layer (7).

13. A method as claimed in claim 1, the method further comprising the steps of:
providing the plurality of absorbent sheets (1) in a collapsed configuration; and
unfolding the plurality of absorbent sheets (1) into an operative configuration, wherein the collapsed configuration has smaller spatial dimensions than the operative configuration.

14. A method as claimed in claim 13, wherein the collapsed configuration is held together by a fastener (8).

15. A method as claimed in claim 1, the method further comprising the step of:
layering the plurality of absorbent sheets (1) upon each other.
